# EUROPEAN PATENT APPLICATION

(11) **EP 4 542 552 A1**
(43) Date of publication of application: **23.04.2025**
(21) Application number: 23824140.0
(22) Date of filing: 05.06.2023
(51) Int. Cl.: G16B 25/20, G16B 50/00, G16H 70/00, G16H 15/00

(54) **METHOD AND DEVICE FOR CREATING TECHNICAL CONSTRUCTION FILE**

(30) Priority: 15.06.2022 KR 20220072827
(71) Applicant: Seegene, Inc., Seoul 05548 (KR)
(72) Inventor: LEE, Kwang Il, Seoul 05232 (KR); GU, Gyeong Mo, Goyang-si Gyeonggi-do 10546 (KR); OH, Hye Jin, Seoul 05318 (KR); KIM, Ju Hwa, Yongin-si Gyeonggi-do 16840 (KR); LEE, Han Byul, Guri-si Gyeonggi-do 11930 (KR); KIM, Han Deul, Gunpo-si Gyeonggi-do 15874 (KR); KOO, Won Jun, Hanam-si Gyeonggi-do 12917 (KR); KANG, Eun Ju, Yeosu-si Jeollanam-do 59720 (KR); KIM, Sun Hyung, Anyang-si Gyeonggi-do 14009 (KR)
(74) Representative: Viering, Jentschura & Partner mbB Patent- und Rechtsanwälte
(86) International application number: PCT/KR2023/007710
(87) International publication number: WO 2023/243922

(57) **Abstract**

A computer-implemented method for generating a technical construction file for a reagent for detecting a target nucleic acid molecule according to one embodiment, includes: providing an input screen for items common to at least two or more detailed performance experiments among a plurality of detailed performance experiments for the reagent for detecting the target nucleic acid molecule, wherein the technical construction file includes two or more detailed performance documents including the at least two or more detailed performance experiments; receiving input of contents for the common items at once; and exporting the common items and the received contents for the common items to the two or more detailed performance documents to generate the two or more detailed performance documents.

## Description

### TECHNICAL FIELD

The present disclosure relates to a method for generating a technical document (technical construction file) for a reagent for detecting a target analyte.

### BACKGROUND ART

There are various methods for amplifying target analytes or target analyte substances, particularly target nucleic acid molecules. For example, these may include polymerase chain reaction (PCR), ligase chain reaction (LCR) (U.S. Patent Nos. 4,683,195 and 4,683,202; PCR Protocols: A Guide to Methods and Applications (Innis et al., eds, 1990)), strand displacement amplification (SDA) (Walker et al. Nucleic Acids Res. 20(7):1691-6 (1992); Walker PCR Methods Appl 3(1):1-6 (1993)), transcription-mediated amplification (Phyffer et al., J. Clin. Microbiol. 34:834-841 (1996); Vuorinen et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatchet et al., Genet. Anal. 15(2):35-40 (1999)), Q-Beta Replicase (Lizardi et al., BiolTechnology 6:1197(1988)), loop-mediated isothermal amplication (LAMP, Y. Mori, H. Kanda and T. Notomi, J. Infect. Chemother, 2013, 19, 404-411), and recombinase polymerase amplication (RPA, J. Li, J. Macdonald and F. von Stetten, Analyst, 2018, 144, 31-67).

The reagents for detecting the target analytes used in the amplification reactions described above are sold as medical devices. In this case, in order to sell the medical devices, the requirements for approval and certification in each country must be met. These requirements are described in technical documents, and a standard technical construction file (TCF) is an example of such technical documents.

The data described in such technical documents may include, for example, data on experimental method and/or experimental results for performance experiments conducted during the development process. Here, a plurality of performance experiments may be conducted depending on the types of performance to be approved/certified. In this case, a plurality of technical documents may be generated to correspond to the plurality of types of performance experiments, respectively.

The technical documents corresponding to the respective performance experiments may include common items. This is because each performance experiment targets the same target analyte. For example, the name and type of the target analyte described in the technical documents may correspond to the common items.

### DETAILED DESCRIPTION OF INVENTION

### TECHNICAL PROBLEMS

The objectives to be achieved in one embodiment include providing a technique for generating the aforementioned technical documents. For example, when generating the aforementioned technical document for each of a plurality of types of performance experiments performed on a specific target analyte, the aforementioned objectives may include ensuring that the common items described above are easily recorded in the plurality of technical documents without human error.

However, problems to be solved by the present disclosure are not limited to the foregoing, and other unmentioned objects would be apparent to one of ordinary skill in the art from the following description.

### TECHNICAL SOLUTION

A computer-implemented method for generating a technical construction file for a reagent for detecting a target nucleic acid molecule according to one embodiment, includes: providing an input screen for items common to at least two or more detailed performance experiments among a plurality of detailed performance experiments for the reagent for detecting the target nucleic acid molecule, wherein the technical construction file includes two or more detailed performance documents including the at least two or more detailed performance experiments; receiving input of contents for the common items at once; and exporting the common items and the received contents for the common items to the two or more detailed performance documents to generate the two or more detailed performance documents.

Further, types of performance to be tested in the plurality of detailed performance experiments include at least one of analytical sensitivity, analytical specificity, reproducibility, repeatability, and stability of the detection reagent.

Further, the items provided through the input screen include at least two of experimental materials, experimental equipment, sample preparation, and experimental methods for the detailed performance experiments.

Further, the item for the sample preparation includes an item for nucleic acid extraction method.

Further, the item for the experimental method includes an item for preparation of amplification reaction.

Further, the item for the experimental materials includes items for organism names of microorganism strains and purchase source information.

Further, the item for preparation of amplification reaction includes
items for volume of each material constituting an amplification reaction mixture, the type of container containing the amplification reaction mixture, and information on PCR protocol.

Further, the items include a type of enzyme.

Further, the method further includes receiving modifications to the received contents, and the modifications are reflected in at least some of the detailed performance documents among the plurality of detailed performance documents.

Further, the items include a first item and a second item, and depending on contents received for the first item, a type of the second item, an input screen for the second item, and/or contents to be received for the second item are dependently changed.

Further, the first item includes a type of enzyme, and the second item includes an amplification protocol used in an amplification reaction.

Further, the technical construction file is a document requiring approval, and includes information about participants involved in the approval and details about each participant.

Further, the participants involved in the approval include an author, a reviewer, and a final approver of the technical construction file.

A computer device for generating a technical construction file according to one embodiment, includes a memory storing at least one instruction; a communication unit configured to perform communication with a database; a display unit; and a processor for executing the at least one instruction to generate a technical construction file of a reagent for detecting a target analyte, wherein the technical construction file includes two or more detailed performance documents including at least two detailed performance experiments, and wherein the processor is configured to: provide an input screen for items common to the at least two detailed performance experiments among a plurality of detailed performance experiments for the reagent for detecting the target nucleic acid molecule; receive input of contents for the common items at once; and export the common items and the received contents for the common items to the at least two detailed performance documents to generate the two or more detailed performance documents.

In a computer-readable recording medium storing a computer program according to one embodiment, the computer program includes instructions for, when executed by one or more processors, causing the one or more processors to perform a method for generating a technical construction file for a reagent for detecting a target analyte in a technical construction file generating device, the technical construction file includes two or more detailed performance documents including at least two or more detailed performance experiments, and the method includes: providing an input screen for items common to two or more detailed performance experiments among a plurality of detailed performance experiments for the reagent for detecting the target nucleic acid molecule; receiving input of contents for the common items at once; and exporting the common items and the received contents for the common items to the two or more detailed performance documents to generate the two or more detailed performance documents.

### EFFECT OF INVENTION

According to one embodiment, a technical document for each of the plurality of types of performance experiments performed on a target analyte can be generated easily and without human error.

### BRIEF DESCRIPTION OF THE DRAWINGS

FIG. 1 illustrates a technical document generating device according to one embodiment and a database connected thereto.
FIG. 2 is a block diagram of the technical document generating device according to one embodiment.
FIG. 3 exemplarily shows items that are common to each of detailed performance documents according to one embodiment.
FIG. 4 is an example diagram showing items preset in the detailed performance documents for different performance experiments according to one embodiment.
FIG. 5 is an example diagram of an input screen for allowing an author using the technical document generating device according to one embodiment to enter items that are common to at least two detailed performance documents.
FIG. 6 is an example diagram of an input screen for entering contents regarding information on Materials and Equipment used in a reagent for detecting a target nucleic acid molecule that is the subj ect of a detailed performance experiment according to one embodiment.
FIG. 7 is an example diagram of an input screen for entering contents regarding sample preparation and experimental method items for a reagent for detecting a target nucleic acid molecule that is the subject of a detailed performance experiment according to one embodiment.
FIG. 8 is an example diagram of an input screen for entering contents regarding experimental method and experimental result items for a reagent for detecting a target nucleic acid molecule according to one embodiment.
FIG. 9 is an example diagram of an input screen for entering content for experimental results according to one embodiment.
FIG. 10 is a flowchart for explaining a method for generating a detailed performance document performed in the technical document generating device according to one embodiment.
FIG. 11 is a flowchart for explaining a method for generating a detailed performance document performed in the technical document generating device according to one embodiment.

### MODE FOR CARRYING OUT THE INVENTION

Advantages and features of the disclosure, and methods for achieving the same may be apparent from the embodiments described below with reference to the accompanying drawings. However, the disclosure is not limited to the embodiments disclosed herein, and various changes may be made thereto. The embodiments disclosed herein are provided only to inform one of ordinary skilled in the art of the category of the disclosure. The disclosure is defined only by the appended claims.

When determined to make the subject matter of the disclosure unclear, the detailed description of known functions or configurations may be skipped. The terms described below are defined considering the functions in embodiments of the disclosure and may be replaced with other terms according to the intention or practice of the user or operator. Therefore, the terms should be defined based on the overall disclosure.

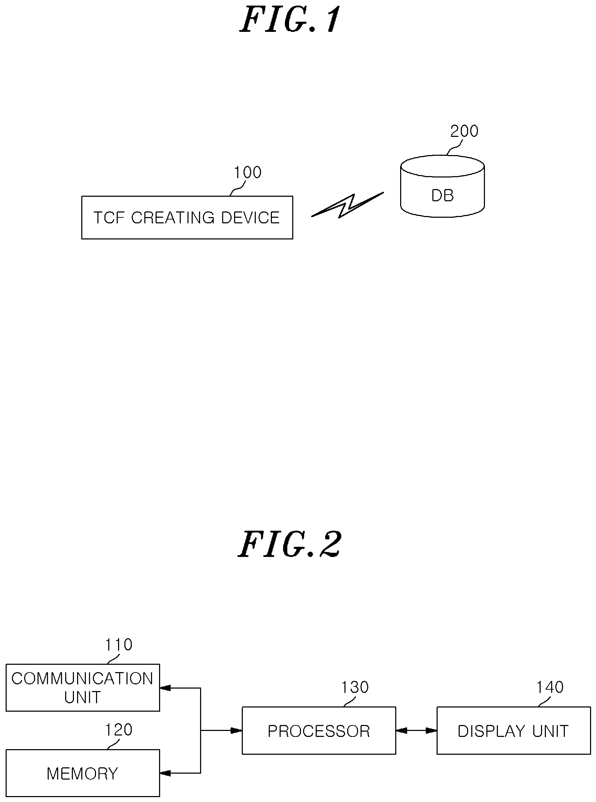

Before explaining FIG. 1, the terms used herein will be described.

The term "target analyte" includes various substances (e.g., biological substances and non-biological substances), which may refer to the same subject as the term "target analyte substance."

Such target analytes may specifically include biological substances, more specifically at least one of nucleic acid molecules (e.g., DNA and RNA), proteins, peptides, carbohydrates, lipids, amino acids, biological compounds, hormones, antibodies, antigens, metabolites, and cells.

The term "sample" refers to biological samples (e.g., cells, tissues, and body fluids) and non-biological samples (e.g., food, water, and soil). Among these, the biological samples may include at least one of, for example, viruses, bacteria, tissues, cells, blood (including whole blood, plasma, and serum), lymph, bone marrow fluid, saliva, sputum, swab, aspiration, milk, urine, stool, eye fluid, semen, brain extract, spinal fluid, joint fluid, thymic fluid, bronchial lavage fluid, ascites, and amniotic fluid. These samples may or may not contain the target analytes described above.

Meanwhile, when the aforementioned target analyte is a nucleic acid molecule or contains a nucleic acid molecule, a nucleic acid extraction process known in the art can be performed on the sample presumed to include the target analyte (see: Sambrook, J. et al., Molecular Cloning. A Laboratory Manual, 3rd ed. Cold Spring Harbor Press (2001)). The nucleic acid extraction process may vary depending on the type of sample. In addition, when the extracted nucleic acid is RNA, a reverse transcription process for synthesizing cDNA may be additionally performed (see: Sambrook, J. et al., Molecular Cloning. A Laboratory Manual, 3rd ed. Cold Spring Harbor Press (2001)).

The term "data set" refers to data obtained from a signal generation reaction for the target analyte using a signal generation means (which will be described later).

In this case, the term "signal generation reaction" means a reaction that generates a signal dependent on properties of a target analyte in a sample, such as activity, amount or presence (or absence) of the target analyte, specifically presence (or absence). Such signal generation reactions include biological reactions and chemical reactions. Among these, biological reactions include genetic analysis processes such as PCR, real-time PCR, isothermal amplification and microarray analysis, immunological analysis processes, and bacterial growth analysis. In addition, chemical reactions include processes that analyze the generation, transformation, or destruction of a chemical substance. According to one embodiment, the signal generation reaction may be a genetic analysis process, or may be a nucleic acid amplification reaction, an enzymatic reaction, or microbial growth.

Meanwhile, the signal generation reaction described above is accompanied by a signal change. Therefore, the progress of the signal generation reaction can be evaluated by measuring the signal change.

In this case, the term "signal" means a measurable output. Furthermore, the measured magnitude or change of the signal serves as an indicator that qualitatively or quantitatively indicates the properties of the target analyte, specifically the presence or absence of the target analyte in the sample.

Examples of indicators include, but are not limited to, fluorescence intensity, luminescence intensity, chemiluminescence intensity, bioluminescence intensity, phosphorescence intensity, charge transfer, voltage, current, power, energy, temperature, viscosity, light scatter, radioactivity intensity, reflectance, transmittance, and absorbance.

The term "signal generating means" as mentioned above refers to a means for providing a signal indicating the properties, specifically the presence or absence, of the target analyte to be analyzed.

Such signal generating means include a label itself or an oligonucleotide to which the label is linked.

Among these, the label includes a fluorescent label, a luminescent label, a chemiluminescent label, an electrochemical label, and a metallic label. The label may be used as is, such as an intercalating dye. Alternatively, the labels may be used in the form of a single label or an interactive dual label including a donor molecule and an acceptor molecule, linked to one or more oligonucleotides.

When using a fluorescent label, the signal value may be expressed as RFU (Relative Fluorescence Unit) values.

The signal generating means may additionally include an enzyme having a nucleic acid cleavage activity to generate a signal (e.g., an enzyme having a 5' nucleic acid cleavage activity or an enzyme having a 3' nucleic acid cleavage activity).

Meanwhile, various methods for generating a signal indicating the presence of a target analyte, particularly a target nucleic acid molecule, using the signal generating means are known. Representative examples may include: TaqManTM probe method (U.S. Patent. No. 5,210,015), Molecular beacon method (Tyagi, Nature Biotechnology, v.14 MARCH 1996), Scorpion method (Whitcombe et al., Nature Biotechnology 17:804-807 (1999)), Sunrise (or Amplifluor) method (Nazarenko et al., Nucleic Acids Research, 25(12):2516-2521 (1997), and U.S. Patent. No. 6,117,635), Lux method (U.S. Patent. No. 7,537,886), CPT (Duck P et al. Biotechniques, 9:142-148 (1990)), LNA method (U.S. Patent. No. 6,977,295), Plexor method (Sherrill CB et al., Journal of the American Chemical Society, 126:4550-4556 (2004)), Hybeacons (D.J. French et al., Molecular and Cellular Probes 13:363-374 (2001) and U.S. Patent. No. 7,348,141), Dual-labeled, self-quenched probe (U.S. Patent. No. 5,876,930), Hybridization probe (Bernard PS et al., Clin Chem 2000, 46, 147-148), PTOCE (PTO cleavage and extension) method (WO 2012/096523), PCE-SH (PTO Cleavage and Extension-Dependent Signaling Oligonucleotide Hybridization) method (WO2013/115442), PCE-NH (PTO Cleavage and Extension-Dependent Non-Hybridization) method (PCT/KR2013/012312), and CER method (WO 2011/037306).

Meanwhile, the term "signal generating reaction" mentioned above may include an amplification reaction of a signal. In this case, the term "amplification reaction" means a reaction that increases or decreases a signal generated by the signal generation means. Specifically, the amplification reaction means a reaction of increasing (or amplifying) a signal generated by the signal generation means depending on the presence of a target analyte.

In such amplification reactions, amplification of a target analyte (e.g., a nucleic acid molecule) may or may not be accompanied. More specifically, the amplification reaction may mean an amplification reaction of a signal accompanied by amplification of a target analyte.

Meanwhile, the data set obtained through the amplification reaction may include amplification cycles.

Here, the term "cycle" refers to a unit of change in a certain condition in a plurality of measurements involving a change in the certain condition. The change in the certain condition may refer to an increase or decrease in temperature, reaction time, number of reactions, concentration, pH, number of replications of a measurement target (e.g., nucleic acid), etc., for example. Accordingly, the cycle may be a time or process cycle, a unit operation cycle, and a reproductive cycle.

More specifically, when a reaction of a certain process is repeated or a reaction is repeated at certain time intervals, the term "cycle" means a single unit of the repetition.

Alternatively, when a certain action is repeated as a reaction progresses, the term "cycle" may mean a single unit of the repetition.

For example, when a nucleic acid amplification reaction is performed, the act of detecting a signal generated at regular time intervals may be repeated, and the cycle may mean a single unit of the repetition. In this case, the cycle may have a unit of time.

For example, in the case of a nucleic acid amplification reaction, one cycle means a reaction including the denaturation phase of the nucleic acid, the primer annealing phase, and the primer extension phase. In this case, a change in a certain condition is an increase in the number of repetitions of the reaction, and a repetition unit of the reaction including the series of phases is set as one cycle. The number of cycles may include the number of reactions or the reaction time.

Meanwhile, the aforementioned target analytes or target analyte substances, especially target nucleic acid molecules, can be amplified using various methods: polymerase chain reaction (PCR), ligase chain reaction (LCR) (U.S. Patent Nos. 4,683,195 and 4,683,202; PCR Protocols: A Guide to Methods and Applications (Innis et al., eds, 1990)), strand displacement amplification (SDA) (Walker et al. Nucleic Acids Res. 20(7):1691-6 (1992); Walker PCR Methods Appl 3(1):1-6 (1993)), transcription-mediated amplification (Phyffer et al., J. Clin. Microbiol. 34:834-841 (1996); Vuorinen et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatchet et al., Genet. Anal. 15(2):35-40 (1999)), Q-Beta Replicase (Lizardi et al., BiolTechnology 6:1197(1988)), loop-mediated isothermal amplication (LAMP, Y. Mori, H. Kanda and T. Notomi, J. Infect. Chemother, 2013, 19, 404-411), and recombinase polymerase amplication (RPA, J. Li, J. Macdonald and F. von Stetten, Analyst, 2018, 144, 31-67).

Meanwhile, the amplification reaction amplifies the signal while amplifying the target analyte (specifically, the target nucleic acid molecule). For example, the amplification reaction is performed according to PCR, specifically real-time PCR, or isothermal amplification reaction (e.g., LAMP or RPA).

Meanwhile, the data set obtained by the signal generation reaction includes a plurality of data points including cycles of the signal generation reaction and signal values in the cycles.

In this case, the term "signal value" means a quantified value of a signal level (e.g., signal intensity) actually measured during the cycles of a signal generation reaction, particularly an amplification reaction based on a certain scale, or a transformed value thereof. The transformed value may include a mathematically processed signal value of the actually measured signal value. Examples of mathematically processed signal values of the actually measured signal value (i.e., the signal value of the raw data set) may include logarithmic values or derivatives.

The term "data point" means a coordinate value that includes a cycle and a signal value. In addition, the term "data" means all information that constitutes a data set. For example, each of the cycle and the signal value of an amplification reaction may be considered data.

Data points obtained by a signal generation reaction, particularly an amplification reaction, may be expressed as coordinate values that can be expressed in a two-dimensional Cartesian coordinate system. In the coordinate values, the X-axis represents the number of cycles, and the Y-axis represents the signal value measured or processed in the corresponding cycle.

The term "data set" means a collection of the data points. For example, the data set may be a collection of data points obtained directly through an amplification reaction performed in the presence of a signal generating means, or may be a modified data set obtained by modifying such a data set. The data set may be some or all of a plurality of data points obtained by the amplification reaction, or modified data points thereof.

Meanwhile, the data set may be a data set obtained by processing multiple data sets. When performing analyses on a plurality of target analytes in a single reaction vessel, in some cases, the data sets for the plurality of target analytes may be obtained by processing data sets obtained from reactions performed in the single reaction vessel. For example, the data sets for the plurality of target analytes in the single reaction vessel may be obtained by processing multiple data sets derived from signals measured at different temperatures.

The aforementioned data set can be plotted, thereby obtaining an amplification curve.

The term "plate" refers to a standard unit in which an amplification reaction is performed in an amplification device, and a basic unit in which data generated after the amplification reaction is stored. Different plates may be plates in which an amplification reaction is performed at different times using the same amplification device, or plates in which an amplification reaction is performed at the same time using different amplification devices.

The plate includes a plurality of reaction wells. The plate may include N x M reaction wells. Typically, the plate includes 12 x 8 or 8 x 12 reaction wells. The reaction wells of the plate may be integrated with the plate or may be in the form of a detachable tube. The plate may be rectangular in shape, but the plate may also be implemented in various shapes, such as circular, trapezoidal, and rhomboidal, as long as it includes one or more reaction wells.

The wells of the plate contain the sample to be analyzed and the reagents necessary for the nucleic acid amplification reaction.

Hereinafter, various embodiments of the present disclosure will be described with reference to the drawings.

FIG. 1 illustrates a technical document generating device 100 according to one embodiment and a database 200 connected thereto. The technical document generating device 100 and the database 200 may be connected to each other through wired or wireless communication. However, FIG. 1 is merely an example, and the idea of the present disclosure is not limited to the configuration shown in FIG. 1. For example, a configuration not illustrated in FIG. 1 may be additionally connected to these components 100 and 200. Alternatively, unlike what is illustrated, the database 200 may be implemented by being included in the computer device 100. However, the following description will be made on the premise that the aforementioned components 100 and 200 are implemented or connected as illustrated in FIG. 1. Hereinafter, each component will be described in detail.

Various types of data are stored in the database 200. The stored data may include various data for generating the technical document described above, but is not limited thereto. For example, as will be discussed later, items to be described in the technical document are stored in the database 200 in the form of files. These items may be categorized and stored according to a plurality of performance experiments to be described later.

Here, in order to sell medical devices such as reagents for detecting target analytes, the requirements for country-specific approval and certifications must be satisfied. Technical documents refer to documents that demonstrate compliance with such country-specific approval and certification requirements. The technical documents include, for example, a standard technical construction file (TCF), but are not limited to this.

The technical document generating device 100 generates the aforementioned technical documents.

The technical document includes various information. Specifically, the technical documents may include experimental information regarding performance experiments performed during the reagent development process, such as at least one of experimental preparation, experimental method, and materials and equipment used in the experiments. In addition, the performance experiment may include a plurality of detailed performance experiments in which the types of performance to be tested are different. Accordingly, the technical documents of the present disclosure may include at least two detailed performance documents. Hereinafter, the process of generating such technical documents will be described.

Specifically, developers need to repeatedly enter the same content for items that are common or typically included in each detailed performance document. During this process, even experienced developers can make human errors such as typos. In addition, if a typo is found, especially if the typo is for a common item in the plurality of detailed performance documents for each performance experiment, developers or document authors need to individually correct each detailed performance document. In other words, the correction process may be cumbersome.

In this case, by using the technical document generating device 100 according to one embodiment, a developer or document author can easily create such technical documents without human error.

Hereinafter, the technical document generating device 100 will be described in more detail.

FIG. 2 is a block diagram of the technical document generating device 100 according to one embodiment.

Referring to FIG. 2, the technical document generating device 100 includes a communication unit 110, a memory 120, a processor 130, and a display unit 140, but the present disclosure is not limited thereto.

First, the communication unit 110 is implemented as a wired or wireless communication module. Through the communication unit 110, the technical document generating device 100 can communicate with the outside. For example, as will be described later, the technical document generating device 100 can transmit content input by an author to the outside through the communication unit 110. In addition, the technical document generating device 100 can also receive experimental information necessary for generating a technical document from the outside, for example, from the database 200 illustrated in FIG. 1.

The memory 140 stores various types of data or information including at least one instruction. The data to be stored may include data received from the database 200 through the communication unit 110 or data processed by the processor 130, and the data or information may include various types of experimental information necessary for generating technical documents.

Meanwhile, although FIG. 2 depicts the memory 120 as a separate component from the processor 130, the memory 120 may be implemented as a single unit with the processor 130. For example, the memory 120 may be a storage such as a cache included inside the processor 130.

Next, the processor 130 may be implemented as a central processing unit (CPU), a graphics processing unit (GPU), a micro controller unit (MCU), or a dedicated processor that performs the methods according to the embodiments. Hereinafter, the processor 130 may collectively refer to a single processor or multiple processors, such as a multi-core processor.

The processor 130 can write data to the memory 120. In addition, the processor 130 can read out and execute instructions stored in the memory 120. For example, the processor 130 can enable the technical document generating device 100 to perform the functions to be described below by executing instructions stored in the memory 120. These functions will be described later.

The display unit 140 may be driven by the processor 130 as follows.

First, the display unit 140 may display information. In addition, the display unit 140 may receive certain information from a user. The display unit 140 may be implemented as a touch screen or a touch pad, or alternatively, may be implemented through a combination of an LCD monitor and a keyboard. In this case, the information may be that transmitted from the outside to the technical document generating device 100 through the communication unit 110 or loaded from the memory 120, but the present disclosure is not limited thereto.

The display unit 140 according to the present disclosure displays a screen for receiving experimental information necessary for generating technical documents. The content or contents included in the screen may be transmitted those from an external source such as the database 200 to the technical document generating device 100 through the communication unit 110 or loaded from the memory 120, but the present disclosure is not limited thereto.

In addition, the display unit 140 provides an input screen to allow the processor 130 to generate a technical document for a reagent for detecting a target analyte.

The technical document is a set of at least two detailed performance documents including at least two detailed performance experiments.

The detailed performance documents correspond to the detailed performance experiments for different types of performance to be tested, respectively. The detailed performance experiment may be a test required to be performed for the approval of a reagent for detecting a target analyte.

Each detailed performance document is a document that describes, after testing the performance of a reagent for detecting a target analyte under various conditions to optimize its performance, the experimental information (experiment preparation, sample preparation, experiment method, materials and equipment used in the experiment, etc.) according to the purpose of each test. Accordingly, there may be at least two detailed performance documents for each product.

Here, the reagent for detecting the target analyte includes a detection composition. In addition, the above-mentioned 'performance optimization' refers to a test process to ensure that each material constituting the detection composition included in the reagent functions properly or is adjusted to function optimally.

In order to optimize the performance of the reagent for detecting the target analyte, tests on detection compositions classified by purpose are required, and experimental information including the test method and the test results during this process should be recorded in the technical document as it reflects the suitability of the reagent.

In one embodiment, the types of performance to be tested in a plurality of detailed performance experiments, classified by purpose, include at least one of analytical sensitivity, analytical specificity, reproducibility, repeatability, and stability of the reagent for detection.

In the present disclosure, a detailed performance document is generated for each type of performance to be tested. The set of a plurality of detailed performance documents is referred to as a technical document.

The technical document is a document that describes information on tests required to be performed for the approval of a reagent for detecting a target analyte in a detailed performance document for each performance experiment.

In this case, even though the technical document for a reagent for detecting a specific target analyte includes detailed performance documents for different performance experiments, there may be common items and content for each detailed performance document as described above.

In such cases, the same content may need to be repeatedly entered for common items in the detailed performance documents, or even standardized items that are usually required may also need to be manually entered repeatedly. However, this process may lead to human errors, such as mistakes or typos.

To this end, the technical document generating device 100 according to the present disclosure enables the processor 130 to provide, on the display unit 140, an input screen for items common to each of detailed performance documents for at least two performance experiments among a plurality of performance experiments for different types of performance to be tested.

Alternatively, in some embodiments, the input screen may provide an input screen for an item corresponding to a detailed performance document for a single performance experiment.

The technical document generating device 100 receives common items and contents for the common items only once through the input screen displayed on the display unit 140, and generates detailed performance documents for at least two performance experiments including the common items and the contents entered for the common items. That is, the technical document generating device 100 exports the information into at least two detailed performance documents to generate two or more detailed performance documents.

As described above, the technical document generating device 100 is intended to provide a user interface to enable more convenient and accurate generation of technical document for a target detection reagent through the input screen.

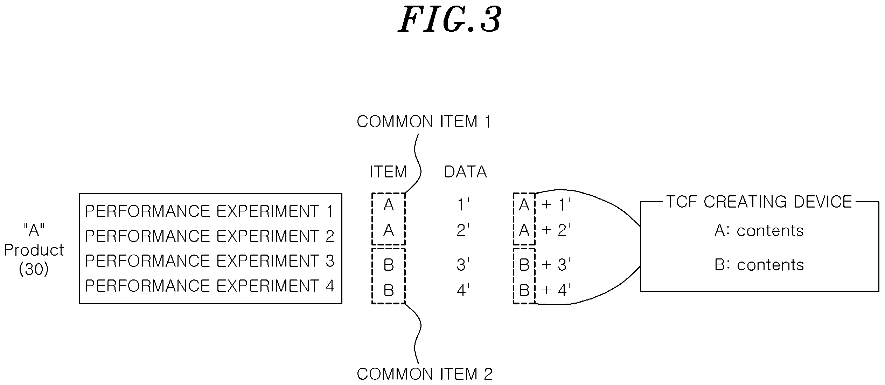

Hereinafter, each functional configuration of the technical document generating device 100 providing such a user interface will be described in more detail with reference to FIGS. 3 to 10.

First, the technical document generating device 100 displays, through the input screen, common items for each of the detailed performance documents for at least two detailed performance experiments among a plurality of detailed performance experiments for different types of performance to be tested. To help understanding, an exemplary implementation where the common items are used is illustrated in FIG. 3.

FIG. 3 illustrates an implementation example of common items for each detailed performance document according to one embodiment. Specifically, referring to FIG. 3, as illustrated, in order to generate a technical document for a specific target analyte detection reagent product 30, different performance experiments 1, 2, 3, 4... n are required. In this case, the number of types of performance experiments is n (where n is a natural number), and the number illustrated in FIG. 3 is merely an example. The performance experiments 1, 2, 3, and 4 may include various test items, among which there are identical items for the detailed performance experiments. For example, item "A" is the identical item in performance experiments 1 and 2, and item "B" is the identical item in performance experiments 3 and 4. As a matter of course, performance experiment 1 may include other non-common items in addition to item "A," and the same applies to performance experiments 2 to 4.

According to the embodiment, item "A" is a common item for performance experiments 1 and 2, and item "B" is a common item for performance experiments 3 and 4.

Since these common items exist in each of the detailed performance experiments, a technical document author conventionally accessed each of the detailed performance documents for each of the plurality of detailed performance experiments and repeatedly described the common items and the same content for them.

However, the technical document generating device 100 according to the present disclosure provides the input screen for items common to each detailed performance experiment, and when content is entered into the items (the items common to each detailed performance document) displayed on the input screen, the items and the entered content therefor are collectively inserted into the detailed performance document for each performance experiment.

In this case, the action of entering content for each item is a single input action that is not repeated more than once. In other words, the action of entering specific content for each item can be unique in the input screen.

For example, the contents, such as an identification number for a product, the names of author/reviewer/approver, a serial number for experimental equipment, and the names of materials used in the experiment, are recorded in each of the plurality of detailed performance documents by a single input action, that is, with just a single entry.

In order to avoid the conventional approach of repeatedly entering common items and their contents for each detailed performance document, the present disclosure identifies items that are commonly repeated in the detailed performance documents and provides them through the input screen. Each of the common items may also be displayed uniquely on the input screen, similar to the content for each item described above.

The common items may be representative names for individual contents to be entered, and may include, for example, Product Name, Cat No, Approval, History, Materials, Equipment list, Nucleic acid extraction, Preparation of PCR reaction, PCR Cycler parameter for Signal Detection, Results, Data Analysis, etc.

Accordingly, the technical document generating device 100 according to the present disclosure generates detailed performance documents to include the common items for each detailed performance document and the content entered for each item.

In this case, the detailed performance document may include not only the common items and the content input for each item through the input screen of the present disclosure, but also unique content individually for each detailed performance document. The unique content for each detailed performance document may be entered for each document through a separate document input means, not through the technical document generating device 100 according to one embodiment, but the present disclosure is not limited thereto.

Since the plurality of performance experiments have different purposes for testing, each performance experiment may have unique items and contents.

As shown in FIG. 3, if the data of performance experiments 1, 2, 3, and 4 are respectively indicated as "1"', "2"', "3"', and "4"', the technical document is a set of, for example, the common item "A" and data 1' of the detailed performance document of performance experiment 1, the common item "A" and data 2' of the detailed performance document of performance experiment 2, the common item "B" and data 3' of the detailed performance document of performance experiment 3, and the common item "B" and data 4'of the detailed performance document of performance experiment 4.

Among these, the technical document generating device 100 according to the present disclosure provides a user interface that provides an input screen for entering the common items "A" and "B" and contents therefor.

When the common items and their contents are entered through the input screen, the technical document generating device 100 according to the present disclosure includes them in the detailed performance documents to generate each detailed performance document.

In one embodiment, the common items and the content entered for each item through the input screen are inserted into a file saved with a specified extension based on the format for items preset for each detailed performance document.

In this case, the preset items for each detailed performance document include, for example, TITLE, Document NO., Revised NO., Revised Date, Objective of test, and Methods of test, and these preset items are in a standardized format for each detailed performance document of a technical document that is applied identically to each product, and the arrangement information such as location, spacing, and line on the document may be identical for each document.

Preset items may be located at the top of each page in the detailed performance document. However, the location is not limited to the above.

Preset items for each page of the detailed performance document may be individually stored as files with specific extensions in the database 200 for each page. Alternatively, they may be stored for each detailed performance document.

The file according to the present disclosure can be generated using a tool known in the art that helps to easily generate documents of various types, for example.

Using the tool, preset items for each page of a detailed performance document are generated, and data fields for inserting common items and content therefor according to the present disclosure are set on the page where the preset items are generated, and the file is then stored in the database 200.

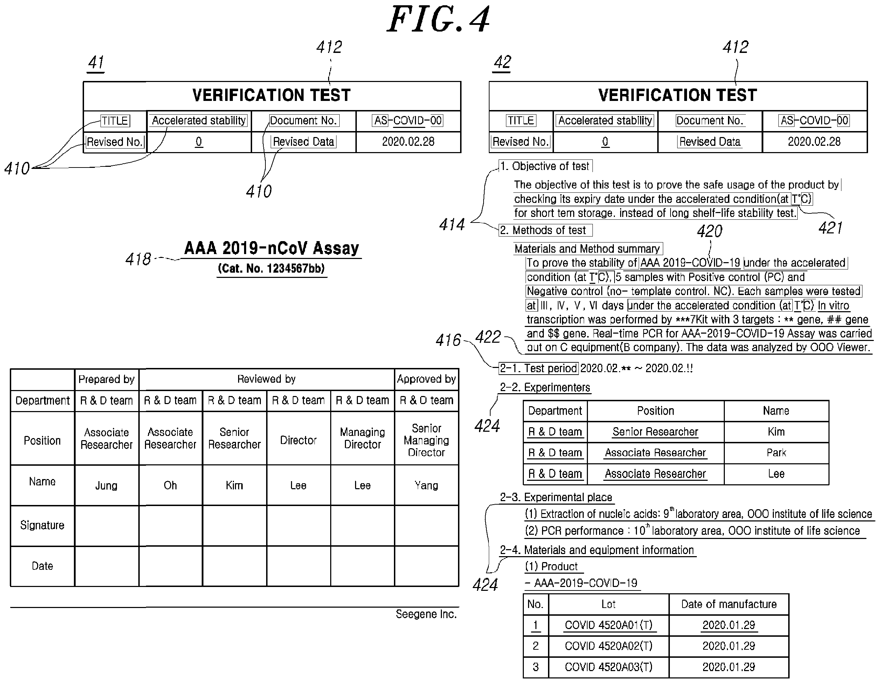

FIG. 4 illustrates the structure of such a file. As shown, in the file stored in the database 200 for generating a technical document according to the present disclosure, the preset items, which are basic frames, are individually stored with specific extensions for each page of the detailed performance document, even if the product changes.

For example, as shown in FIG. 4, preset items 410, 414, and 416 are specified at the top of each page 41 and 42 of the detailed performance document of the Accelerated stability performance experiment for "VERIFCATION TEST" 412 and are respectively saved as individual files with different names.

Below the preset items 410, 414, 416 (box) on each page 41 and 42, data fields 418, 420, 422, and 424 are set for inserting content for common items (including AAA 2019-COVID-19, Cat. No. COVID10243X, COVID10244Y underlined), so that the content entered through the input screen is inserted to be located in the set data fields.

Thereafter, contents for common items in each detailed performance experiment, entered through the input screen according to the present disclosure, can be inserted into the corresponding location for each page based on the data fields set in the file.

Accordingly, the TCF author can easily generate a detailed performance document by inserting common items and contents therefor entered through the input screen of the present disclosure into the file stored in the database 200 using the technical document generating device 100 without the need for coding each item with program codes or accessing each document to generate the detailed performance document.

Since the technical document generating device 100 of the present disclosure inserts contents for the common items for each detailed performance document into the file, the process of entering contents for the common items through the input screen according to the embodiment may be referred to as technical document generation. Alternatively, the process in which the contents for the common items are included in the detailed performance document (inserted into the file) through the input screen may only be referred to as technical document generation. However, the present disclosure is not limited to the above. In this case, in order to allow the contents to be included in the detailed performance document (inserted into the file), "Complete" of the input screen described below may be used.

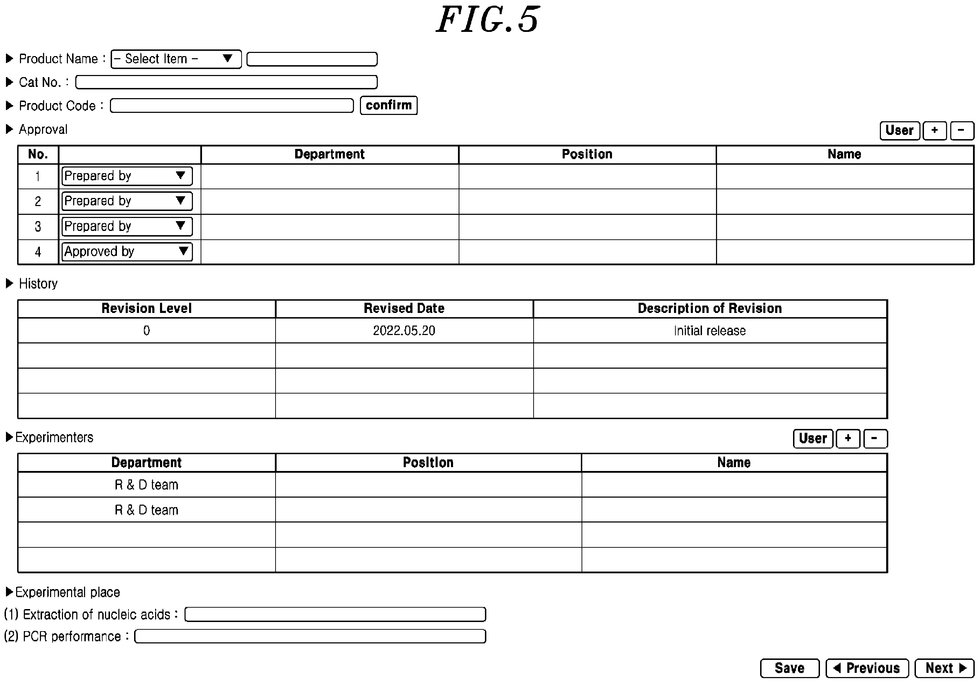

Hereinafter, an implementation example of receiving contents for common items to each performance experiment through the input screen in the technical document generating device 100 that provides such a technical document generation UI will be described in more detail with reference to FIGS. 5 to 9.

FIG. 5 illustrates the input screen that allows a technical document author using the technical document generating device 100 according to one embodiment to input items that are common to at least two detailed performance documents.

On the input screen of FIG. 5, in particular, a product overview for a product that is the subject of a detailed performance experiment can be entered as content.

As shown in FIG. 5, the common product overview items include Product Name, Cat No, and Product code. The Product Name may be selected from a preset product list through a submenu display. The selected product name becomes the contents of the Product Name item, which can be inserted into the corresponding location in the file through a "Complete" selection action to be described later.

In the present disclosure, when entering content for the "Product code" item, a duplication check of code can be performed by selecting "confirm." In this case, the Product code is a symbol used in barcodes, which is widely used for product identification, and when "confirm" is selected, the Product code already entered in a given DB is compared with the Product code entered on the current screen to check whether the Product code (entered on the current screen) duplicates any existing codes.

In the "Approval" item, information (department, position, and name) of the person who generated, reviewed, and approved the document is entered as content.

In addition, the generation date, revision date, grade, etc. of the detailed performance document are entered as content for the History item.

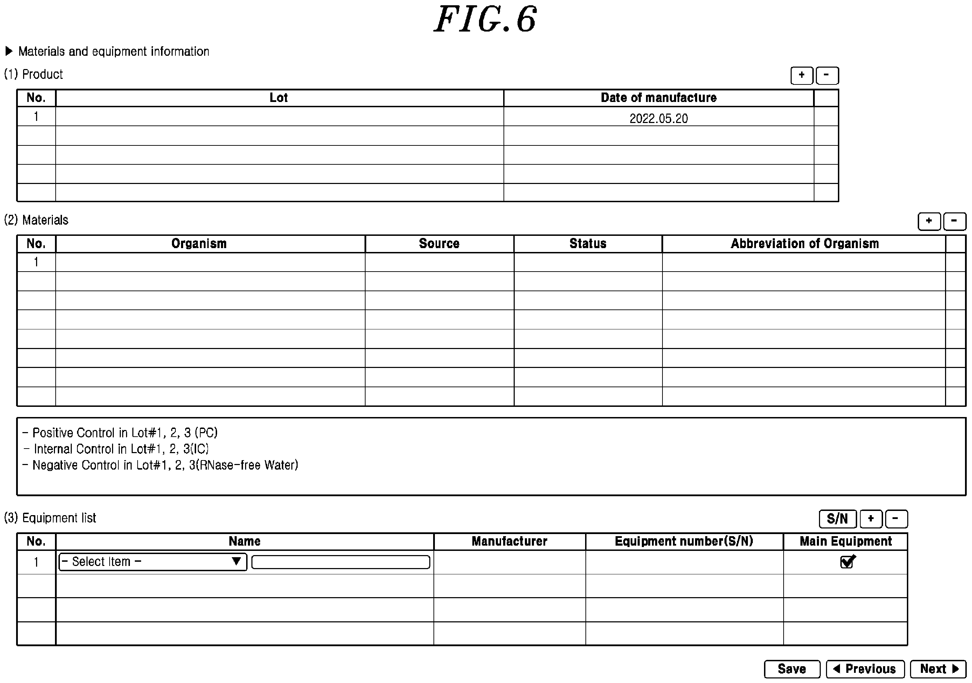

The contents entered for each item are inserted into the data fields set in the aforementioned file at the corresponding location for each detailed performance document. FIG. 6 illustrates an exemplary input screen for entering contents regarding information on Materials and Equipment used in a reagent for detecting a target nucleic acid molecule that is the subject of a detailed performance experiment according to one embodiment.

The input screen illustrated in FIG. 6 includes information (manufacturing date, etc.) on a reagent for detecting a target nucleic acid molecule, the names of materials constituting the reagent, such as oligonucleotides (primers and probes), buffers, enzymes used in amplification reactions, and salts, and organism names for microorganism strains and purchase source information for each material, and contents for Source, Status, Abbreviation, etc. can be entered in the input screen. In each table, rows can be added or deleted using the "+" and "-" buttons at the top of the table.

In this case, the items (No, Lot, Date of manufacture, Organism, Source, Status, Abbreviation of Organism) described in each table shown in FIG. 6 are set as items in the table in advance along with the items set in advance in the above-described file according to the embodiment, and the content for each item in each table can be inserted in the corresponding location by setting the data field based on the table set in the above-described file.

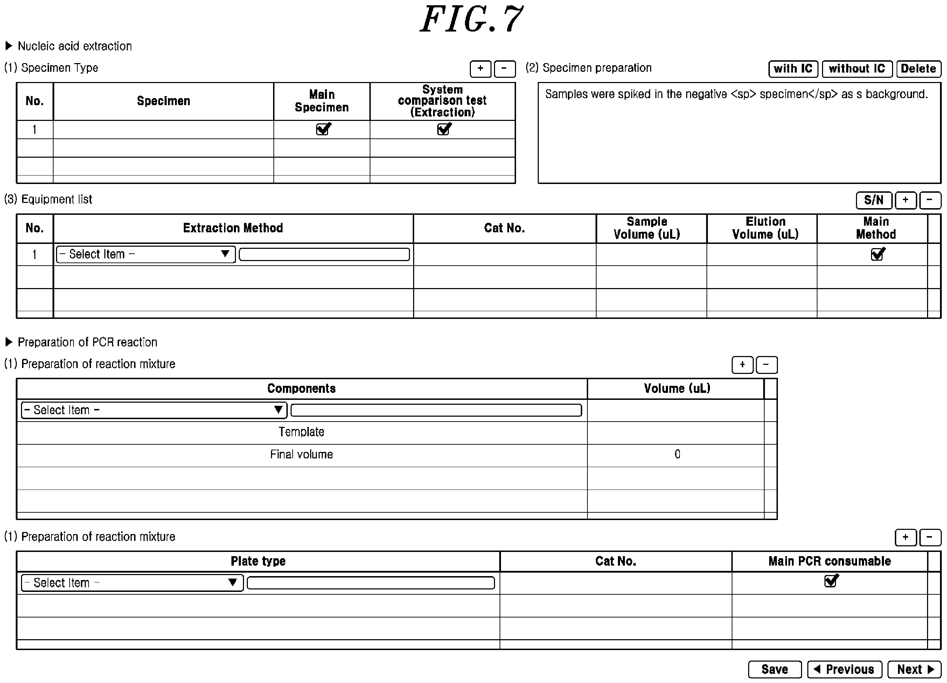

FIG. 7 illustrates an exemplary input screen for entering contents regarding sample preparation and experimental method items for a reagent for detecting a target nucleic acid molecule that is the subject of a detailed performance experiment according to one embodiment.

In the input screen illustrated in FIG. 7, contents for the nucleic acid extraction method included in the items for sample preparation for detailed performance experiment for target nucleic acid molecule detection and contents for the Preparation of PCR reaction included in the experimental method item can be entered.

In order to perform detection of a target nucleic acid molecule using the reagent for detecting the target nucleic acid molecule according to the present disclosure, it is first necessary to remove a substance that inhibits an amplification reaction from a biological sample and then extract the nucleic acid. The extracted nucleic acid is used to prepare an amplification reaction mixture.

Since information on the process of preparing a detection composition, such as the nucleic acid extract preparation and the amplification reaction mixture preparation, is basic content for items described in a technical document, in the input screen according to the present disclosure, contents for sample preparation items can be entered through the screen shown in FIG. 7.

Referring to FIG. 7, description items for the nucleic acid extraction method in the technical document include Specimen Type, Specimen Preparation, and Extraction of nucleic acids. In the Specimen Type item, the specimen name and whether it is a main specimen or not may be entered.

In the Specimen Preparation item, depending on whether the specimen is endogenous or exogenous, information about the case of the inherent presence or absence of IC of the specimen may be entered by selecting the "with IC" and "without IC" buttons.

In the Extraction of nucleic acids item, information about the extraction equipment for extracting nucleic acids and the operation method of the extraction equipment, the number of the extraction reagent, Sample Volume, and Elution Volume.

Next, in the input screen shown in FIG. 7, as the experimental method item, content regarding Preparation of PCR reaction for the reagent for detecting target nucleic acid molecules may be entered.

In one embodiment, the Preparation of PCR reaction item includes items for information on the volume of each material constituting the amplification reaction mixture and the type of container containing the amplification reaction mixture.

In the item for the volume of each material constituting the amplification reaction mixture, the contents of enzyme volume, template volume, and total volume of the enzyme and template volume may be entered.

The amplification protocol includes the time, temperature, and number of cycles during target nucleic acid molecule amplification. For example, the amplification protocol starts at 98°C/3min, and then performs 39 cycles of 98°C/10s and 58°C/20s. After starting at 95°C/10s and 65°C/10s, the melting curve is performed from 65°C to 95°C with the temperature increasing by 0.3°C per cycle. In the input screen of the present disclosure, enzymes for each amplification protocol used in the amplification reaction can be preset by type, according to embodiments. Accordingly, a user using the input screen according to the present disclosure can select the most suitable enzyme based on the amplification reaction TAT (turnaround time) and enter it as the content for the material of the Preparation of PCR reaction item. In this case, the enzyme may be empirically selected based on repeated experiments, or may be preset based on repeated experiments.

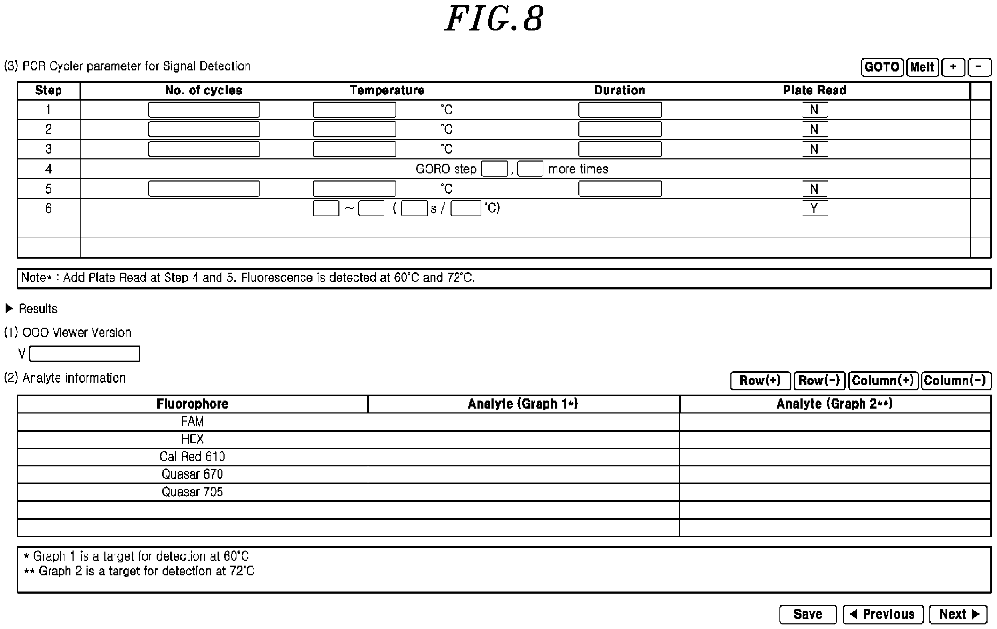

In the present disclosure, the items include a first item and a second item, and according to one embodiment, in the input screen, depending on the content entered for the first item, the type of the second item, the input screen for the second item, or the content to be entered for the second item may be dependently changed. Here, the first item includes the type of enzyme, and the second item includes the amplification protocol used for the amplification reaction, but the present disclosure is not limited thereto. Hereinafter, this will be described in detail with reference to FIG. 8.

FIG. 8 is an example diagram of an input screen for entering contents for an experimental method item of a reagent for detecting a target nucleic acid molecule according to one embodiment.

In the input screen shown in FIG. 8, information about the PCR Cycler parameter for Signal Detection, that is, information about the PCR protocol can be entered.

In the PCR Cycler parameter for Signal Detection, which is an item for the experimental method, information about the PCR protocol, that is, contents including the temperature, reaction time, and number of reactions when performing the amplification reaction can be entered step by step. In each step, whether Plate Read has been performed can be entered.

In this case, in the input screen according to the present disclosure, the amplification protocol, i.e., PCR protocol, may be changed dependently depending on the type of enzyme among the contents entered in the Preparation of PCR reaction item through the input screen of FIG. 7. As described above, since the most suitable enzyme can be determined based on repeated experiments according to the temperature and time conditions of the PCR protocol, the PCR Cycler parameter for Signal Detection item may be changed according to the determined enzyme.

Next, in FIG. 8, contents for the experimental result items can be entered.

In the experimental result items according to the present disclosure, the version of the equipment or SW that performs the analysis of the experimental results can be entered, and contents for the Analyte information item can be entered. In the Analyte information item, the names of the analytes used at both low and high temperatures for each channel can be entered as the contents.

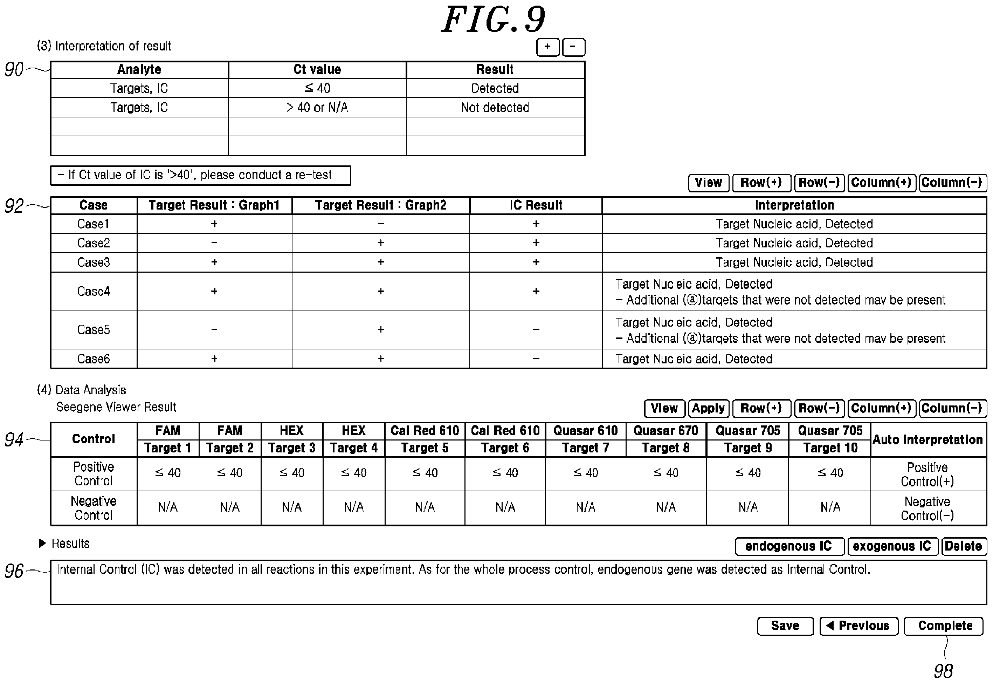

FIG. 9 illustrates an exemplary input screen for entering content for experimental result items according to one embodiment.

As shown in FIG. 9, in the input screen according to the present disclosure, interpretation information indicating that detection was made at or above/below a Ct (Threshold Cycle) value for the corresponding analyte and IC can be entered as content.

This is because the technical document requires interpretation information, that is, the criteria for determining the presence or absence of a target analyte using a reagent for detecting a target nucleic acid molecule.

For example, in the input screen of FIG. 9, the interpretation information that the presence or absence of the target analyte was determined based on the fact that in the first row of Table 90, the target analytes, IC were detected at a Ct value of 40 or higher, and the targets, IC were not detected at a Ct value of 40 or lower as in the second row, can be entered as the content of the interpretation of result item.

Table 92 shows interpretation information for the contents entered for each case based on how the target and IC are read positive/negative, and the "view" button at the top of the table can be used to display interpretation information for all contents entered for each case.

In Table 94, the detection results of the target analytes analyzed by equipment or SW that performs analysis on experimental results can be entered as content.

To this end, Table 94 can display the names of target nucleic acid molecules for which channel-specific readings have been performed. In this case, the names of target nucleic acid molecules for each channel can be applied identically by using the "Apply" button without the need to re-enter the analyte names for each channel previously entered when entering the contents for the Analyte information item on the input screen illustrated in FIG. 8.

In addition, in Table 94, the positive/negative reading criteria by name for the target nucleic acid molecule for which the channel-specific readings have been performed can be entered.

Table 96 is for the description of the internal control, and the content for the IC experiment results can be entered. For example, the "endogenous" or "exogenous" button at the top of Table 96 can be selected, and the detection results for IC in the amplification reaction for the selected "endogenous" or "exogenous" can be entered as the content.

The contents for the common items described through the above description of the input screen in FIGS. 5 to 9 are generated into at least two detailed performance documents through the "Complete" button 98.

As described above, with reference to FIGS. 5 to 9, the implementation example of entering the contents of the common items for each detailed performance experiment through the input screen including a plurality of pages according to one embodiment.

For convenience of explanation, the page-by-page input screens illustrated in FIGS. 5 to 9 display items that are common to each detailed performance experiment based on a typical technical document, and contents for the items are entered; however, the input screens of the present disclosure are not limited thereto.

The input screen according to the present disclosure can display the description items of the detailed performance document for the detailed performance experiment in various ways according to the embodiments.

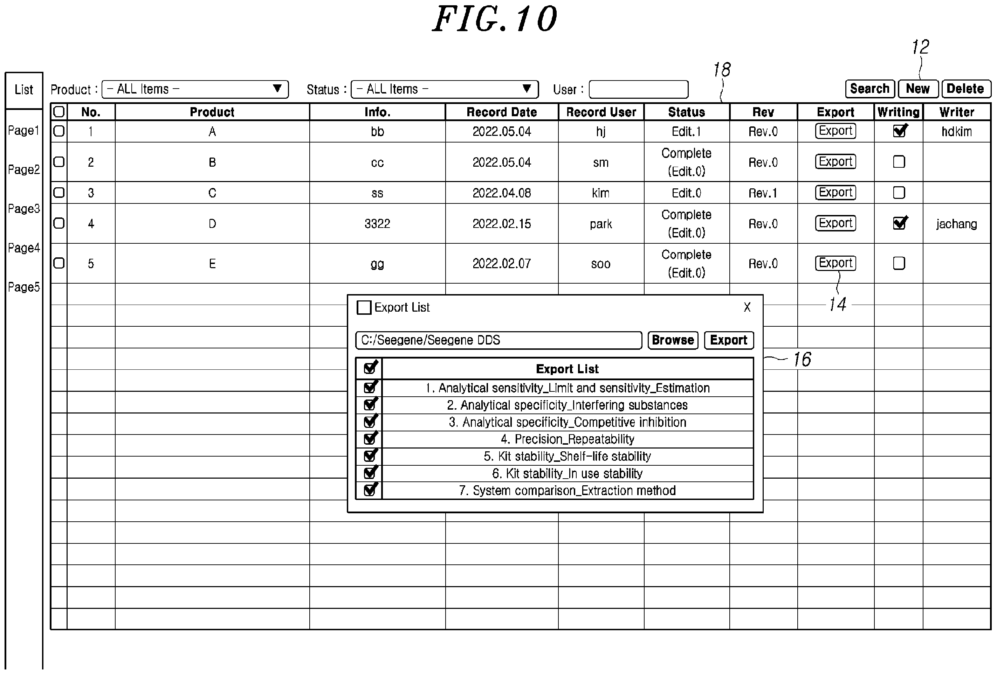

FIG. 10 is an example diagram showing an implementation example of an initial screen 10 of the technical document generating device 100 according to one embodiment.

The initial screen 10 illustrated in FIG. 10 may display a list of products for which technical document generation has been completed by generating detailed performance documents for each performance experiment using the contents entered through the input screen.

In addition, the initial screen 10 may display a selection button "New" 12 for providing multiple page-by-page input screens for generating a technical document for a new product.

In the initial screen 10 according to one embodiment, it is possible to determine whether to export each detailed performance document for each of the plurality of detailed performance experiments. This can be done by selecting "Export" 14 shown in FIG. 10.

Specifically, when the "Complete" button 98 illustrated in FIG. 9 is clicked, the generation of a technical document corresponding to the content entered for each item through the input screens of FIGS. 5 to 9 is completed, and the completed technical document can be displayed with a product name in the list for each product on the initial screen 10 as mentioned above. In this case, the "Export" button 14 is activated. When the activated "Export" button 14 is selected, a list of performance experiments 16 according to one embodiment may be displayed in a pop-up form.

As mentioned above, the types of performance to be tested in the plurality of detailed performance experiments include at least one of analytical sensitivity, analytical specificity, reproducibility, repeatability, and stability of the detection reagent.

According to one embodiment, as shown in reference numeral 16 of FIG. 10, a check button may be assigned to each of the plurality of detailed performance experiments. Depending on whether each check button is selected, the export of the detailed performance document of the corresponding performance experiment may be determined. For example, depending on whether the check button is selected, only certain detailed performance documents among the plurality of detailed performance experiments may be selectively exported.

This export is intended to insert common items for each detailed performance document into the aforementioned file to complete the generation of the technical document, save the completed technical document, specify the storage location, and modify some of the detailed performance documents.

In the technical document generating device 100 according to the present disclosure, the contents entered for each item through input screens including a plurality of pages can be modified as needed.

According to one embodiment, the modifications may be applied to at least some of the detailed performance documents.

According to one embodiment of the present disclosure, in the case where a modification is required in a specific detailed performance document, a user can select the page where the modification is required on the input screens including the plurality of pages and reflect the modification. Only the detailed performance document for the performance experiment including the modified page can be separately exported through the check button indicated in reference numeral 16 of FIG. 10, and the previously exported version of the corresponding detailed performance document is updated to reflect the modification made in the newly exported detailed performance document.

Based on this, according to one embodiment, the initial screen 10 illustrated in FIG. 10 may display a list of products for which technical document generation has been completed, along with the status of technical document generation for each product.

For example, the status information displayed in the Status item 18 in the table illustrated in FIG. 10 may include "Edit" and "Complete." "Edit" may mean that editing (modification) of the corresponding detailed performance documents is possible, and "Complete" may mean that the technical document generation for the corresponding detailed performance documents has been completed and can be viewed only in a read-only mode.

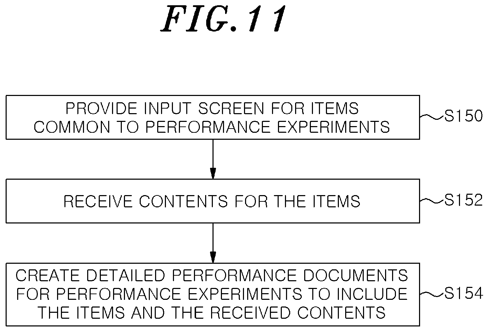

FIG. 11 is a flowchart for explaining a method for generating a technical document performed in the technical document generating device according to one embodiment.

In the present disclosure, the technical document generating device performs a method for generating a technical document of a reagent for detecting a target nucleic acid molecule, wherein the technical document includes at least two detailed performance documents.

Referring to FIG. 11, in step S150, the technical document generating device provides an input screen for items common to at least two performance experiments among a plurality of performance experiments in which the types of performance to be tested are different.

In step S152, the technical document generating device receives contents for the items.

In step S154, the technical document generating device generates detailed performance documents for at least two performance experiments to include the items and the input contents.

Since the operation of each step illustrated in FIG. 11 is identical to the function of the technical document generating device 100 described above, the descriptions of FIGS. 1 to 10 described above will be referred.

As described above, the method for generating a technical document performed in the technical document generating device 100 according to the present disclosure can provide the input screen for items common to at least two performance experiments, among a plurality of detailed performance documents for each performance experiment that constitute the technical document. The contents for the items entered through the input screen are inserted into a file in which preset items of the technical document are stored for each detailed performance document. Through this process, the technical document is completed.

The block diagrams and the combinations of blocks and steps of the flowchart provided in the present disclosure may be performed by a computer program.

The computer program includes instructions for, when executed by one or more processors, causing the one or more processors to perform the method for generating a technical document for a reagent for detecting a target analyte in the technical document generating device, wherein the technical document includes at least two detailed performance documents, and the method includes the steps of: providing an input screen for items common to at least two performance experiments among a plurality of performance experiments in which the types of performance to be tested are different; receiving contents for the items; and generating detailed performance documents for the at least two performance experiments to include the items and the received contents./

The computer program may also be stored on a computer-readable recording medium to implement functionality in a specific manner.

Further, when an element "comprises," "includes," or "has" another element, the element may further include, but rather than excluding, the other element, and the terms "comprise," "include," and "have" should be appreciated as not excluding the possibility of presence or adding one or more features, numbers, steps, operations, elements, parts, or combinations thereof. All the scientific and technical terms as used herein may be the same in meaning as those commonly appreciated by a skilled artisan in the art unless defined otherwise. It will be further understood that terms, such as those defined dictionaries, should be interpreted as having a meaning that is consistent with their meaning in the context of the relevant art and will not be interpreted in an idealized or overly formal sense unless expressly so defined herein.

The above-described embodiments are merely examples, and it will be appreciated by one of ordinary skill in the art various changes may be made thereto without departing from the scope of the disclosure. Accordingly, the embodiments set forth herein are provided for illustrative purposes, but not to limit the scope of the disclosure, and should be appreciated that the scope of the disclosure is not limited by the embodiments. The scope of the disclosure should be construed by the following claims, and all technical spirits within equivalents thereof should be interpreted to belong to the scope of the disclosure.

While embodiments of the disclosure have been described above, it will be apparent to one of ordinary skill in the art that the specific techniques are merely preferred embodiments and the scope of the disclosure is not limited thereto. Thus, the scope of the disclosure is defined by the appended claims and equivalents thereof.

## Claims

1. A computer-implemented method for generating a technical construction file for a reagent for detecting a target nucleic acid molecule, the method comprising:
providing an input screen for items common to at least two or more detailed performance experiments among a plurality of detailed performance experiments for the reagent for detecting the target nucleic acid molecule, wherein the technical construction file includes two or more detailed performance documents including the at least two or more detailed performance experiments;
receiving input of contents for the common items at once; and
exporting the common items and the received contents for the common items to the two or more detailed performance documents to generate the two or more detailed performance documents.

2. The method of claim 1, wherein types of performance to be tested in the plurality of detailed performance experiments include at least one of analytical sensitivity, analytical specificity, reproducibility, repeatability, and stability of the detection reagent.

3. The method of claim 1, wherein the items provided through the input screen include at least two of experimental materials, experimental equipment, sample preparation, and experimental methods for the detailed performance experiments.

4. The method of claim 3, wherein the item for the sample preparation includes an item for nucleic acid extraction method.

5. The method of claim 3, wherein the item for the experimental method includes an item for preparation of amplification reaction.

6. The method of claim 3, wherein the item for the experimental materials includes items for organism names of microorganism strains and purchase source information.

7. The method of claim 5, wherein the item for preparation of amplification reaction includes items for volume of each material constituting an amplification reaction mixture, the type of container containing the amplification reaction mixture, and information on PCR protocol.

8. The method of claim 1, wherein the items include a type of enzyme.

9. The method of claim 1, further comprising receiving modifications to the received contents,
wherein the modifications are reflected in at least some of the detailed performance documents among the plurality of detailed performance documents.

10. The method of claim 1, wherein the items include a first item and a second item, and
wherein depending on contents received for the first item, a type of the second item, an input screen for the second item, and/or contents to be received for the second item are dependently changed.

11. The method of claim 10, wherein the first item includes a type of enzyme, and
the second item includes an amplification protocol used in an amplification reaction.

12. The method of claim 1, wherein the technical construction file is a document requiring approval, and includes information about participants involved in the approval and details about each participant.

13. The method of claim 11, wherein the participants involved in the signing include an author, a reviewer, and a final approver of the technical construction file.

14. A computer device for generating a technical construction file, comprising:
a memory storing at least one instruction;
a communication unit configured to perform communication with a database;
a display unit; and
a processor for executing the at least one instruction to generate a technical construction file of a reagent for detecting a target analyte,
wherein the technical construction file includes two or more detailed performance documents including at least two detailed performance experiments, and
wherein the processor is configured to:
provide an input screen for items common to the at least two detailed performance experiments among a plurality of detailed performance experiments for the reagent for detecting the target nucleic acid molecule;
receive input of contents for the common items at once; and
export the common items and the received contents for the common items to the at least two detailed performance documents to generate the two or more detailed performance documents.

15. A computer-readable recording medium storing a computer program, wherein:
the computer program includes instructions for, when executed by one or more processors, causing the one or more processors to perform a method for generating a technical construction file for a reagent for detecting a target analyte in a technical construction file generating device, wherein the technical construction file includes two or more detailed performance documents including at least two or more detailed performance experiments, and
wherein the method includes:
providing an input screen for items common to two or more detailed performance experiments among a plurality of detailed performance experiments for the reagent for detecting the target nucleic acid molecule;
receiving input of contents for the common items at once; and
exporting the common items and the received contents for the common items to the two or more detailed performance documents to generate the two or more detailed performance documents.
